# EUROPEAN PATENT SPECIFICATION

(11) **EP 4 452 238 B1**
(45) Date of publication and mention of the grant of the patent: **04.02.2026**
(21) Application number: 22843885.9
(22) Date of filing: 22.12.2022
(51) Int. Cl.: A61K 9/51, A61K 9/1271, A61K 47/68, A61K 47/69, A61K 31/7105, A61K 9/1272

(54) **METHOD FOR THE PREPARATION OF HYBRID POLYMERIC NANOPARTICLES FOR THE RELEASE OF OLIGONUCLEOTIDE DRUGS IN PHARMACOLOGICAL THERAPIES FOR REGENERATIVE, CURATIVE, AND PREVENTIVE PURPOSES**
VERFAHREN ZUR HERSTELLUNG VON HYBRIDEN POLYMERNANOPARTIKELN ZUR FREISETZUNG VON OLIGONUKLEOTIDARZNEIMITTELN IN PHARMAKOLOGISCHEN THERAPIEN FÜR REGENERATIVE, HEILENDE UND PRÄVENTIVE ZWECKE
PROCÉDÉ DE PRÉPARATION DE NANOPARTICULES POLYMÈRES HYBRIDES POUR LA LIBÉRATION DE MÉDICAMENTS OLIGONUCLÉOTIDIQUES DANS DES THÉRAPIES PHARMACOLOGIQUES À DES FINS DE RÉGÉNÉRATION, DE TRAITEMENT ET DE PRÉVENTION

(30) Priority: 23.12.2021 IT 202100032393
(43) Date of publication of application: 30.10.2024
(73) Proprietor: Politecnico Di Torino, 10129 Torino (IT); Università Degli Studi Di Torino, 10124 Torino (IT)
(72) Inventor: CHIONO, Valeria, 10129 TORINO (IT); MATTU, Clara, 10129 TORINO (IT); NICOLETTI, Letizia, 10129 TORINO (IT); PAOLETTI, Camilla, 10129 TORINO (IT); ARPICCO, Silvia Maria Angela, 10124 TORINO (IT); STELLA, Barbara, 10124 TORINO (IT)
(74) Representative: Bianchetti & Minoja with Trevisan & Cuonzo IPS SRL
(86) International application number: PCT/IB2022/062678
(87) International publication number: WO 2023/119222

(56) References cited:
- WO-A2-2010/142660
- US-A1- 2021 330 597
- JINJUN SHI ET AL: "Differentially Charged Hollow Core/Shell Lipid-Polymer-Lipid Hybrid Nanoparticles for Small Interfering RNA Delivery", ANGEWANDTE CHEMIE, WILEY - V C H VERLAG GMBH & CO. KGAA, DE, vol. 123, no. 31, 22 June 2011 (2011-06-22), pages 7165 - 7169, XP071349875, ISSN: 0044-8249, DOI: 10.1002/ANGE.201101554

## Description

The invention has for object hybrid nanoparticles consisting of a polymeric component encapsulating cationic liposomes and/or lipoplexes comprising a cationic lipid and a helper lipid.

### Background art

Drugs based on nucleic acids are difficult to be administered since:
a) They degrade easily in contact with biologic fluids by nuclease-catalyzed hydrolysis;
b) They do not accumulate in target tissues even when they are released locally or reach the desired site, due to their negative charge preventing the internalization of the oligonucleotide agent by the cells;
c) Differently from the common drugs based on "small molecules," which can be simply delivered at the target tissues, drugs based on nucleic acids require an effective intracellular release.

To overcome the issue of oligonucleotide drug release, different nanotechnologies have been proposed, comprising: (i) lipid nanoparticles, (ii) polymeric nanoparticles, (iii) inorganic nanoparticles, (iv) technology with nano-needles, (v) viral vectors.

Lipid nanoparticles applied for the release of oligonucleotides (for example, miRNAs, siRNAs, mRNAs) combine different types of lipids in order to obtain the desired characteristics.

For example, cationic lipids are widely used at physiological pH, as they are able to effectively complex the oligonucleotide agents. However, they often generate toxic effects on the cells. Cationic lipid nanoparticles were also produced able to encapsulate oligonucleotide agents and other drugs, such as doxorubicin (Sci. Rep. 6, 2016 https://doi.org/10.1038/SREP35223) and paclitaxel [Cancer Med. 6 (2017) 651-661]. US10555910 describes lipid nanoparticles specific for mRNA inhibitors.

However, main drawbacks are represented by the cytotoxicity of the cationic lipids, and their non-specific interactions with serum proteins, making the particles highly unstable. Cytotoxicity is related to the activation of the immune system with the activation of the complement and the coagulation pathways, and the stimulation of cytokine production.

This limitation was overcome through the introduction of neutral lipid particles based on ionizable lipids, which take a positive charge at a slightly acid pH only (thus allowing complexation with RNA), while are neutral at physiological pH, resulting more biocompatible. Generally, lipids with amino moieties having an acid dissociation constant (pKa) of about ~ 6.5 are used. Nanoparticles are obtained by rapidly mixing lipid solutions in ethanol together with aqueous solutions of the oligonucleotide agents in water at low pH (pH 4), which may partially degrade the encapsulated agent. The so produced nanoparticles have a low surface charge at physiological conditions, are non-cytotoxic and nonimmunogenic. Generally, the lipid nanoparticles based on ionizable lipids at low pH contain also (i) a helper lipid, allowing a better liposome formation and promoting interaction with the cell membrane, (ii) cholesterol allowing a better integration of the different lipid components due to its adjuvant function and (iii) a pegylated lipid, limiting the opsonizations by serum proteins and the reticuloendothelial clearance. However, as a drawback, the presence of poly(ethylene glycol) (PEG) coating may limit the interactions with the target cells and intracellular release. This issue can be overcome using pegylated lipids with short acyl chain C14, which are rapidly released *in vivo* [Pharmaceutics. 11 (2019). https://doi.org/10.3390/PHARMACEUTICS11080360].

The relative composition in ionizable lipid, helper lipid, cholesterol, and pegylated lipid influences the efficacy of the lipid nanoparticles and requires an optimization for each application and modality of administration. Furthermore, the chosen lipid type, size and surface charge are very important since they influence the *in vivo* behavior of the lipid nanoparticles.

Particularly, more than 300 ionizable lipids were designed and synthetized identifying the structure-activity relationship [Nat. Nanotechnol. 2019 1412. 14 (2019) 1084-1087]. The particle activity was related to the acid dissociation constant of the cationic ionizable lipid whose optimal value was equal to pKa ≈ 6.4. Deviations from this pKa even of only 0.5 units caused a reduction in efficacy of up to 100 times.

In 2018 the Food and Drug Administration (FDA) and The European Commission (EC) approved Patisiran (ALN-TTR02), a type of lipid nanoparticle (ionizable at low pH) for the release of short interfering RNA (siRNA) for the treatment of polyneuropathies induced by hereditarian amyloidosis.

The most recent example of lipid nanoparticles releasing RNA molecules is represented by Covid-19 BioNTech/Pfizer and Moderna vaccines, releasing a specific ribonucleic acid (mRNA).

However, the use of lipid nanoparticles is limited by instability in biological environment/aqueous solution resulting in poor storage stability: therefore, freezing and use of stabilizers (for example, saccharose) are required.

Another limitation of lipid particles is the non-specific release or the systemic accumulation of oligonucleotide agents. To surface functionalize the particles and ensure a targeted action, the use of lipid agents pre-modified with ligands for cellular targeting is preferred, in order to avoid post-preparation functionalization, which can be critical due to the poor stability of the lipid particles. Therefore, for each surface functionalization, the preparation process has to be optimized, which limits its versatility.

Cationic polymeric nanoparticles for the release of oligonucleotide agents have been proposed, frequently using poly(ethyleneimine) (PEI). It is possible to obtain PEI-based particles, with low and high molecular weight, both with linear and branched polymer structure [Polyethyleneimines for siRNA and miRNA delivery *in vivo,* Wiley Interdiscip. Rev. Nanomed. Nanobiotechnol. 5 (2013) 484-501]. The main drawbacks are cytotoxicity and low transfection efficiency. The combination of PEI with PEG or poly-lysine (PL) reduces cytotoxicity.

An alternative is represented by chitosan particles (EP 2397123), however they require an acidic pH for their preparation, which can degrade the oligonucleotide agent, and have a low transfection efficiency.

Polyamidoamine (PMAM)-based dendrimers have also been proposed, as biodegradable hyper-branched polymers with high transfection efficiency and able to encapsulate hydrophilic and hydrophobic drugs. The main limitation is related to their potential cytotoxic effects [Mater. Today. 18 (2015) 565-572]. Also, polymeric micelles have been reported consisting of polymers with hydrophilic and hydrophobic blocks, allowing the encapsulation of agents with different surface wettability properties.

Additionally, hybrid polymeric nanoparticles have been described containing a hydrophobic polymeric component, such as poly(lactic-co-glycolic acid), poly(lactic acid), polycaprolactone, etc., typically forming the core of the nanoparticle, and a cationic polymeric or lipid component, constituting the outer shell of the nanoparticle. They are produced by a nano-precipitation process allowing the deposition of the cationic component on the particle surface, complexed with the oligonucleotide agent. It is possible to co-encapsulate hydrophobic agents in the core of the hybrid particle. As exposed on the surface, the oligonucleotide is quickly released. Furthermore, the arrangement of components makes surface functionalization of nanoparticles for precision medicine difficult.

Synthetic polymer-cationic component hybrid nanoparticles (US 9549901) able to encapsulate oligonucleotides and other hydrophobic agents have also been prepared through a double-emulsion process (water in oil in water). In this case, the cationic component complexed to the oligonucleotide agent forms the core of the nanoparticle, while the synthetic polymer is exposed on the surface part of the nanoparticle. Surface functionalization of these nanoparticles can be performed using various strategies.

However, the preparation method of the nanoparticles is laborious and time consuming, as it consists of two emulsion steps; furthermore, multiple washings are required, as surfactants with limited biocompatibility are used. Finally, it makes use of high volumes. Overall, the exploitation at industrial level of the double-emulsion process for the preparation of hybrid polymeric nanoparticles is complex.

US 8193334 describes the preparation of hybrid polymeric nanoparticles by two steps: first, inverse micelles based on an amphiphilic (for example, lipidic) component, containing nucleic acids, are prepared in an organic solvent miscible with water (for example, tetrahydrofuran, THF). Then, the synthetic polymer is solubilized in this solvent. Such solution is dropped into water, obtaining the nano-precipitation of the mixture of the synthetic polymer and inverse micelles containing nucleic acids. The reported encapsulation efficacy, particularly of siRNA, is of 18-30%. For the surface functionalization of such nanoparticles, the use of functionalized amphiphilic (for example, lipidic) and polymeric components has been described, since they both may be exposed on the nanoparticle surface.

US 2021/330597 A1 describes polymeric nanoparticles prepared by a double-emulsion process, comprising one or more nano-complexes between a lipid-conjugated cationic polymer and a nucleic acid, and a polymeric shell based on poly(lactic acid-co-glycolic acid)-poly(ethylene glycol) (PLGA-PEG). The weight percentage of the cationic polymer, calculated with respect to the amount of cationic polymer and PLGA-PEG, is comprised between 20% and 55%. The nanoparticle preparation method requires an emulsifying agent, such as poly(vinyl alcohol), and a time of 3 hours to complete solvent evaporation, followed by two final steps of washing/filtration lasting 50 minutes each.

Jinjun Shi et al. [Angewandte Chemie Vol 123 n. 31, 7165-7169] describe nanoparticles consisting of three layers (lipid-polymer-lipid), prepared by double-emulsion and solvent evaporation process. Specifically, the external lipids are 1,2-distearoyl-sn-glycero-3-phosphoethanolamine-N-methoxy(polyethyleneglycol) (DSPE-PEG) and lecithin, polymer is PLGA and, finally, the internal lipid is ethyl phosphocholine (EPC) 14:1. Nanoparticles have a siRNA encapsulation efficiency of 78-82%.

WO 2010/142660 A2 describes microparticles based on a hydrophobic biodegradable polymer (preferably PLGA), covering a cationic lipid complexed to a siRNA, and the method to obtain them. The microparticles have a size comprised between 500 nm and 20 microns (preferably between 2-20 microns) and are prepared by a water in oil in water double-emulsion process, with the use of an emulsifying agent in the second emulsion (oil in water). Furthermore, the component present at higher amount is the cationic lipid, in fact the polymer: siRNA: cationic lipid mass ratio is equal to 5:25:50.

The main methods for the preparation of polymeric nanoparticles able to release oligonucleotides are described in the review published in J. Control. Release, 313 (2019) 80-95. The same review also describes inorganic biocompatible nanoparticles with controllable size and morphology, based on inorganic materials, such as gold, calcium phosphate, silica, and iron oxides.

The nanoparticles (NPs) based on iron oxide (Fe₃O₄) are commonly used as contrast agents for magnetic resonance imaging (MRI). Furthermore, when combined with cationic components, they allow the encapsulation of oligonucleotide agents.

Silica NPs and mesoporous silica (MSP) NPs are nanostructures based on silica having high biocompatibility and stability. They can encapsulate oligonucleotide agents and other drugs and can be surface functionalized with specific molecules for targeted delivery to cells.

Gold nanoparticles (Au NPs) have been used for drug release due to their biocompatibility, ease of functionalization, and ability to obtain them with the desired size and shape. Au-NP surface is usually functionalized with thiol or amine groups to allow the entrapment of the oligonucleotide agents.

Calcium phosphate nanoparticles (CaP NPs), used for about 40 years to encapsulate and deliver oligonucleotides, are cheap, non-toxic, bioresorbable, and easily synthesized. A drawback of such nanoparticles is the low encapsulation efficiency (10-20%) and the difficulty to co-encapsulate other drug types.

The main drawback of some inorganic nanoparticles is the non-degradability and possible accumulation *in vivo,* with consequent potential long-term side effects. A limitation common to all inorganic nanoparticles is the limited encapsulation efficacy.

The use of viral vectors, for example derived from *Retrovirus, Lentivirus, Adenovirus, Herpes virus* and others, for the delivery of oligo- or poly-nucleotides provides advantages, such as cellular transfection efficacy, but includes safety issues for the patient, due to the risks for inflammatory reactions or oncogenesis (for integrating viral vectors). Furthermore, the viral vectors do not allow a targeted action, even if they can show a higher selectivity towards some kinds of cells.

### DESCRIPTION OF THE INVENTION

It has now been found that it is possible to obtain drug-releasing hybrid polymeric nanoparticles, able to efficiently encapsulate oligonucleotides (alone or in combination even with hydrophobic drugs), by a fast, simple, efficient, and scalable process, ensuring a controlled and sustained delivery of the oligonucleotide agents.

The hybrid polymeric nanoparticles of the invention are biocompatible and can be easily functionalized on their surface by a fast, simple, and versatile process which allows their tailoring to the different needs of precision medicine. The nanoparticles of the present invention allow to protect the encapsulated drug from degradation and to efficiently convey it to the target cells by exploiting a specific surface functionalization.

According to the present invention, these and other advantages are achieved by hybrid nanoparticles consisting of a polymeric component encapsulating cationic liposomes and/or lipoplexes comprising a cationic lipid and a helper lipid, in which the polymeric phase is at least 80% by weight with respect to the overall materials of the nanoparticles.

The nanoparticles of the invention are prepared through a nano-precipitation step of the polymeric solution (possibly containing hydrophobic drugs) in an aqueous dispersion of liposomes and/or lipoplexes encapsulating oligonucleotide agents and/or hydrophobic drugs, allowing to expose the polymeric phase on the surface

This allows to efficiently protect the oligonucleotide agents from degradation and to deliver them in a sustained and controlled manner over time. Furthermore, the presence of the synthetic polymer on the surface of the nanoparticles allows their efficient and versatile functionalization and confers them stability during the storage period.

The nanoparticles can be stored for at least one month in aqueous suspension without the aid of stabilizing agents, at a temperature of 4 °C. Alternatively, the nanoparticles can be frozen and thawed to use, recovering their characteristics in aqueous suspension by adding suitable cryo-protective agents. Otherwise, nanoparticles can be frozen and freeze-dried and before use their suspension can be reconstituted with retained physicochemical and functional characteristics always by adding cryo-protective agents.

Furthermore, the nanoparticles have an *in vivo* stability ranging from some days to weeks, depending on their chemical composition, allowing a controlled, sustained, and total release of the encapsulated active ingredients.

The preparation of the nanoparticles involves the nano-precipitation of a polymeric solution into a dispersion of cationic liposomes or lipoplexes, comprising a cationic lipid and a helper lipid, typically by dropping a polymeric solution in water-miscible organic solvents into an aqueous dispersion of liposomes or lipoplexes under stirring. The water-miscible organic solvents are evaporated in a rotary vacuum evaporator. Examples of water-miscible organic solvents comprise alcohols, ketones, acetonitrile, dimethyl sulfoxide, preferably acetone.

Nano-precipitation can be advantageously carried out in a microfluidic device.

The preparation does not require potentially toxic additives, surfactants, or emulsifiers and the production process is efficient, showing a yield higher than 95% (generally equal to 97%), calculated as weight percentage of the product (nanoparticles) with respect to the weight of the used materials. Volumes of the solvents required for the preparation are limited, favoring process scalability. The preparation of the hybrid nanoparticles requires on average less than 1 hour.

The hybrid polymeric nanoparticles based on synthetic polymers and lipid components combine the characteristics of both: the biomimicry of the lipid components and the stability and versatility of the synthetic polymers. The hybrid nanoparticles, object of the invention, represent a new platform for precision medicine, as they are able to efficiently encapsulate oligonucleotides (by the cationic lipid components), possibly in combination with hydrophobic or weakly hydrophilic drugs (by the polymeric component) and to deliver them to the target cells exploiting specific surface functionalization. The process conditions are mild and therefore preserve the functionality of the oligonucleotide agents. The release profile of the oligonucleotides and any other co-encapsulated drugs is more controlled and sustained than for lipidic nanoparticles.

Hence, hybrid nanoparticles allow to decrease the systemic toxicity of therapeutic agents, improving their release and requiring fewer administrations than, for example, lipid nanoparticles. Furthermore, the higher stability compared to lipid nanoparticles allows them to be stored in suspension at a temperature of 4 °C for at least 30 days, without changing their physicochemical characteristics (including encapsulation efficiency).

The versatility of the process ensures wide uses to satisfy different therapeutic needs, for example in the treatment of tumors, in the regenerative cardiovascular, hepatic, and renal medicine, in the treatment of neuromuscular diseases, and in mRNA vaccines.

The nanoparticles of the present invention differ from those described in US 8193334, both for a higher encapsulation efficiency of oligonucleotides (within 96-100% range, therefore higher than 18-30% reported in US 8193334), and for the possibility to encapsulate not only oligonucleotide agents but also hydrophobic or moderately hydrophilic compounds. Furthermore, the preparation process is completely different: according to the present invention, the nano-precipitation of the solution of synthetic polymer occurs on the dispersion containing the pre-formed lipoplexes, allowing the synthetic polymer to cover them. Therefore, the method of the present invention is faster (having one step less), more efficient, and allows to obtain particles which externally expose the synthetic polymer.

### DETAILED DESCRIPTION OF THE INVENTION

The polymeric component used for the preparation of the nanoparticles of the invention can be a single polymer or a blend of more polymers.

The polymeric components are selected from:
(i) polyesters such as poly(lactic-co-glycolic acid) (PLGA), poly(glycolic acid) (PGA), poly(lactic acid) (PLA), polycaprolactone (PCL), poly(lactide-co-caprolactone) (PLCL);
(ii) polyesters from the class of the poly(hydroxy alkanoates), such as poly(hydroxy butyrate) (PHB) and the copolymer poly(hydroxy butyrate-co-hydroxy valerate) (PHBHV);
(iii) poly(propylene oxide) (PPO);
(iv) polymers and copolymers containing methacrylate groups such as poly(methyl methacrylate) (PMMA), poly(ethyl methacrylate) (PEMA) and poly(butyl methacrylate) (PBMA);
(v) poly(cyanoacrylates);
(vi) copolymers comprising the polymers (i) and/or (ii) and/or (iii) and/or (iv) and/or (v), poly(ethylene glycol) PEG and/or (polyethylene oxide) PEO as building blocks;
(vii) polyurethanes or other synthetic copolymers which contain the above-mentioned polymers as building blocks;
viii) natural polymers such as zein, cellulose, lignin, and starch;
ix) modified natural polymers to make them water insoluble (such as butyl glyceryl pectin).

The copolymer poly(lactic-co-glycolic acid) is widely used for its biocompatibility and biodegradability.

The cationic lipid comprises a hydrophilic head with permanent positive charge, for example a hydrophilic head containing ammonium groups.

Examples of cationic lipids comprise [2-(2,3-didodecyloxypropyl)-hydroxyethyl] ammonium bromide (DE), 1,2-di-O-octadecenyl-3-trimethylammonium propane (DOTMA), 1,2-dioleoyloxy-3-[trimethylammonium]-propane (DOTAP), dimethyl-dioctadecyl ammonium bromide (DDAB), cetyl-trimethyl ammonium bromide (CTAB); cationic derivatives of cholesterol such as bis-guanidinium-tren-cholesterol (BGTC), 2,3-dioleyloxy-N-[2-(spermincarboxamido)ethyl]-N,N-dimethyl-1-propaneamino trifluoroacetate (DOSPA). The claims demand that the cationic lipid is selected from [2-(2,3-didodecyloxypropyl)-hydroxyethyl] ammonium bromide, 1,2-di-O-octadecenyl-3-trimethylammonium propane, 1,2-dioleoyloxy-3-[trimethylammonium]-propane, dimethyl-dioctadecyl ammonium bromide, cetyl-trimethyl ammonium bromide, 2,3-dioleyloxy-N-[2-(sperminecarboxamido)ethyl]-N,N-dimethyl-1-propane amino trifluoroacetate, bis-guanidinium-tren-cholesterol.

The percentage by weight of the lipids (cationic lipid and helper lipid) with respect to the total of the nanoparticles can range from 1 to 20%.

The helper lipid consists of one or more phospholipids selected from phosphatidylethanolamine, phosphatidylcholine, cholesterol, and derivatives thereof. The percentages by weight of the helper lipid with respect to the cationic lipid can range from 1 to 50%. The weight ratio between cationic lipid and helper lipid is 50:50.

The size of the nanoparticles can range from 80 to 500 nm. A preferred aspect of the invention consists of nanoparticles containing lipoplexes, comprising a polynucleotide selected from microRNAs, siRNAs, messenger RNAs, plasmid DNAs and, antisense oligonucleotides.

The encapsulation efficiency of the polynucleotides is between 96 and 100%. The hybrid nanoparticle system of the invention is able to release 100% of the loaded active agents and to sustain the release, particularly of RNA, for 7-9 days. The system is stable under storage condition at 4 °C for at least one month.

Alternatively, or in addition to polynucleotides, the nanoparticles can also be loaded with hydrophobic or slightly hydrophilic drugs dispersed in the cationic liposomes or in the polymer.

In the nanoparticles of the invention, the polymer can be surface functionalized with one or more ligands, such as antibodies, antigen-binding antibody fragments (Fab), aptamers, peptides, carbohydrates, small molecules, or a combination of the preceding.

The invention will now be described in greater detail in the following Examples and in the accompanying Figures.

### DESCRIPTION OF THE FIGURES

Figure 1. DLS analysis of the PLGA/DE-DOPE/NegmiR and DE-DOPE/NegmiR nanoparticles in Milli-Q water.
Figure 2. CryoTEM analysis of the PLGA/DE-DOPE/NegmiR nanoparticles.
Figure 3. miRNA encapsulation efficiency of PLGA/DE-DOPE/NegmiR and DE-DOPE/NegmiR nanoparticles.
Figure 4. Storage stability in Milli-Q water at 4 °C of PLGA/DE-DOPE/NegmiR nanoparticles, assessed by DLS analysis.
Figure 5. Storage stability in Milli-Q water and PBS (phosphate buffered saline solution) at 4 °C of PLGA/DE-DOPE/NegmiR nanoparticles, assessed by DLS analysis.
Figure 6. Storage stability in Milli-Q water and DMEM+FBS at 4 °C of PLGA/DE-DOPE/NegmiR nanoparticles, assessed by DLS analysis.
Figure 7. miRNA (NegmiR) release from PLGA/DE-DOPE/NegmiR nanoparticles, assessed by Qubit fluorometric analysis, in dynamic conditions at 37 °C in Milli-Q water.
Figure 8. Cytofluorimetric analysis of adult human atrial cardiac fibroblasts (AHCFs, control) and AHCFs treated with hybrid polymeric nanoparticles based on PLGA-Cy5. The left panel reports the fluorescence of the cells (y axis) vs cellular forward scatter (x axis). The right panel reports the fluorescence of the cells (x axis) vs the cellular count (y axis).
Figure 9. The image on the left shows the expression of intracellular miR-1 after cell transfection with PLGA/DE-DOPE/miRNA nanoparticles respect to control (non-transfected AHCFs) at 48 hours from transfection. The image on the right shows the expression of the target of miR-1 (Twf-1) at 48 hours from transfection with respect to control (non-transfected AHCFs).
Figure 10. Hydrodynamic diameter, zeta potential, and encapsulation efficiency of PLGA/DE-DOPE/miRNA nanoparticles loaded with NegmiR and miRcombo. DLS analysis was performed in Milli-Q water.
Figure 11. Stability of PLGA/DE-DOPE/miRcombo nanoparticles in DMEM-FBS culture medium assessed by DLS analysis.
Figure 12. Stability of PLGA/DE-DOPE/miRcombo nanoparticles in Milli-Q water at 4 °C assessed by DLS analysis.
Figure 13. *In vitro* release of miRcombo in Milli-Q water and in PBS, in dynamic conditions at 37 °C.
Figure 14. Cell viability by resazurin assay performed on AHCFs treated with PLGA/DE-DOPE/miRNA nanoparticles (containing negmiR or miRcombo) at 24, 48, and 72 hours from transfection. The cells (5.5 x 10³) were treated with PLGA/DE-DOPE/miRNA nanoparticles (containing negmiR or miRcombo, 25 nM final). Values are reported respect to control (non-transfected AHCF cells).
Figure 15. Live/Dead assay performed on AHCFs treated with PLGA/DE-DOPE/miRNA nanoparticles (containing negmiR or miRcombo) at 72 hours from transfection. The cells (5.5 x 10³) were treated with PLGA/DE-DOPE/miRNA nanoparticles (containing negmiR or miRcombo, 25 nM final).
Figure 16. Evaluation of the expression of the single microRNA (miR-1, miR-133, miR-208 and miR-499) in AHCFs treated with PLGA/DE-DOPE/miRNA nanoparticles (containing negmiR or miRcombo) at 48 hours from transfection by Droplet Digital PCR. The expression levels of the single microRNAs are reported in fold-change respect to negmiR control.
Figure 17. Direct cardiac reprogramming efficiency through evaluation of the expression of TNNT2 in AHCFs treated with miRcombo using DharmaFECT^{®}/miRNA and PLGA/DE-DOPE/miRNA at 15 days from transfection by Droplet Digital PCR. The expression levels of TNNT2 are related to GAPDH expression (used as house-keeper internal gene).
Figure 18. Hydrodynamic diameter and zeta potential of PLGA/DE-DOPE/miRNA and PLGA-PEG/PLGA/DE-DOPE/miRNA nanoparticles loaded with negmiR. The DLS analysis was performed in Milli-Q water.
Figure 19. Hydrodynamic diameter and zeta potential of PLGA-PEG/PLGA/DE-DOPE/negmiR and IgG/PLGA-PEG/PLGA/DE-DOPE/negmiR nanoparticles. DLS analysis was performed in Milli-Q water.
Figure 20. Fourier infrared spectroscopy analysis (FTIR) of PLGA/DE-DOPE/negmiR nanoparticles (a), PLGA-PEG powder (b), both used as controls, PLGA-PEG/PLGA/DE-DOPE/negmiR nanoparticles (c), and IgG/PLGA-PEG/PLGA/DE-DOPE/negmiR nanoparticles (d).
Figure 21: Cell viability and cytotoxicity assessed on AHCFs treated with PLGA-PEG/PLGA/DE-DOPE/negmiR and IgG/PLGA-PEG/PLGA/DE-DOPE/negmiR nanoparticles at 24 hours from transfection. The cells (5.5 x 10³) were treated with PLGA-PEG/PLGA/DE-DOPE/negmiR and IgG/PLGA-PEG/PLGA/DE-DOPE/negmiR nanoparticles at different concentrations (corresponding at 25, 18.75, 12.5, and 6.25 nM of miRNA). Values are reported relative to the control (non-transfected AHCFs).

### EXAMPLE 1: nanoparticle preparation

PLGA polymer and [2-(2,3-didodecyloxypropyl)-hydroxyethyl] ammonium bromide (DE) cationic lipid and L-α-dioleoyl phosphatidylethanolamine (DOPE) helper lipid were used. However, the method is applicable to any other type of polymer and other lipid combinations, maintaining a polymer content greater than or equal to 80% by weight.

PLGA is solubilized in acetone (1 mg/mL). A volume of 1 mL of DE-DOPE/miRNA lipoplexes was poured in a glass test tube and stirred (about 900-1000 rpm).

Then, the starting PLGA solution, diluted at a concentration equal to 127 µg/mL, was dropped into the lipoplex suspension under magnetic stirring and the system was left under stirring for 30 min. Subsequently, acetone was removed by evaporation. By this method, a volume of 1 mL of PLGA/DE-DOPE/miRNA nanoparticles was obtained. Initially, NegmiR and miR-1 were encapsulated as miRNAs. Then, 4 miRNAs (miRcombo: miR-1, 133, 208, 499) were encapsulated simultaneously. Using the same method, siRNAs can be encapsulated, obtaining similar size, zeta potential, and encapsulation efficacy as for miRNAs. For larger oligonucleotides, such as mRNAs, the N/P ratio between the lipid amine groups and mRNA phosphate groups needs to be optimized.

The PLGA/DE-DOPE/miRNA nanoparticles are functionalized on their surface with a ligand (antibody, antibody fragment, peptide, or aptamer) specific for the adult human cardiac fibroblasts. The functionalization is performed by click chemistry, specifically by copper-catalyzed Cu(I) Huisgen azido-alkyne cycloaddition. The functionalization reaction comprises four steps:
1) functionalization of the PLGA polymer by reaction of carbodiimides with modified PEG with an azide (PEG-N₃), obtaining PLGA-PEG-N₃ (or use of a commercially available PLGA-PEG-N₃ copolymer);
2) preparation of the PLGA-PEG-N₃/DE-DOPE/miRNA nanoparticles by nano-precipitation;
3) functionalization of the ligand with propargyl-PEG-NHS (NHS: N-hydroxy succinimide), obtaining the molecule propargyl-PEG-ligand;
4) conjugation of the propargyl-PEG-ligand bearing an alkyne group, to the azido groups exposed by PLGA-PEG-N₃/DE-DOPE/miRNA nanoparticles.

This kind of functionalization can be applied also for other therapeutic purposes, changing the ligand type.

### EXAMPLE 2: characterization of the nanoparticles

### a) Hydrodynamic size and Z-potential

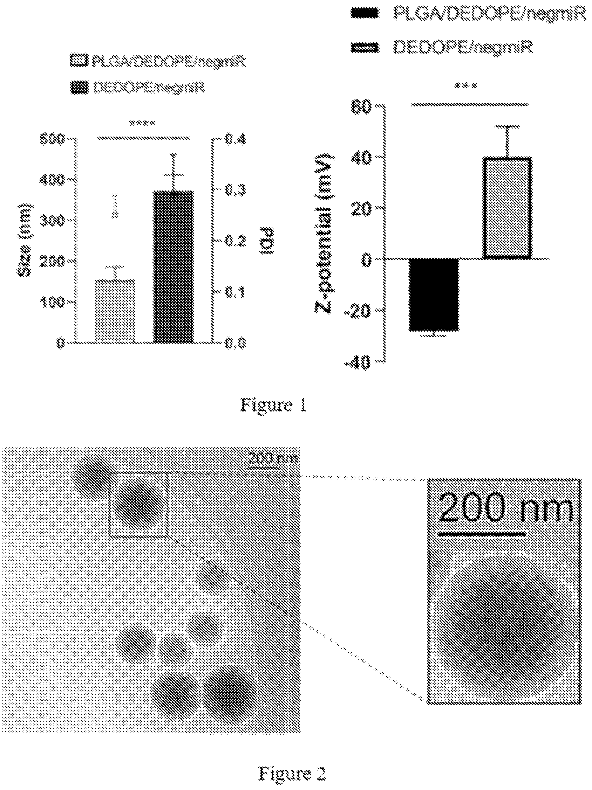

The hybrid PLGA/DE-DOPE/miRNA nanoparticles in Milli-Q water show a hydrodynamic size of 153 nm significatively lower than that of DE-DOPE/NegmiR lipoplexes (372 nm) prepared as control. The Z-potential takes a negative value of -28 mV for PLGA/DE-DOPE/miRNA nanoparticles due to the presence of PLGA on the surface. The DE-DOPE/NegmiR lipoplexes have a positive Z-potential (40 mV) (Figure 1).

DLS analysis of PLGA/DE-DOPE/miRNA nanoparticles in different media showed similar results which are reported in Table 1. This demonstrates the stability of the nanoparticles in contact with different media.

**Table 1. DLS analysis of PLGA/DE-DOPE/NegmiR nanoparticles in different suspension media (PBS: phosphate buffered saline; Dulbecco's Modified Eagle Medium (DMEM)+ Fetal Bovine Serum (FBS): culture medium with fetal bovine serum).**

| **PLGA/DE-DOPE/miRNA NP** | **Size (nm)** | **PDI** | **Z-potential (mV)** |
|---|---|---|---|
| Milli-Q water | 153 ± 33 | 0.25 ± 0.04 | -28 ± 2 |
| PBS | 161 ± 53 | 0.20 ± 0.02 | -31 ± 3 |
| DMEM+FBS | 172 ± 52 | 0.26 ± 0.04 | -28 ± 2 |

### b) Morphological analysis of the nanoparticles

CryoTEM images (Figure 2) show areas at different density suggesting the deposition of PLGA to cover the lipoplexes. The size is nanometric and the particles are separated from each other and do not form aggregates.

### c) Encapsulation efficacy of oligonucleotides (miRNA), EE%

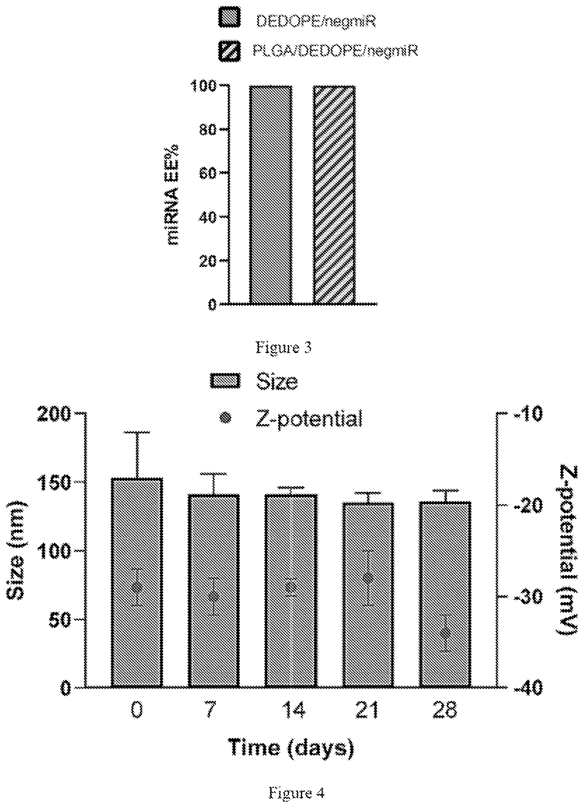

The encapsulation efficacy of a model miRNA (NegmiR) was 99% for PLGA/DE-DOPE/NegmiR nanoparticles and the DE-DOPE/NegmiR lipoplexes (Figure 3). These data were derived by analyzing the supernatants after the nano-precipitation process using Qubit fluorometric technique.

### d) Preparation yield of the hybrid nanoparticles

The yield of the production process (calculated as the weight of the produced nanoparticles respect to the weight of the materials used in the process) was 97±1% for PLGA/DE-DOPE/NegmiR hybrid polymeric nanoparticles.

### e) Storage stability in suspension at 4 °C in Milli-Q water

The hydrodynamic diameter and Z-potential are stable up to at least 28 days of incubation at 4 °C in suspension in Milli-Q water (Figure 4).

### f) Storage stability at 37 °C in Milli-O water and PBS up to 48 h

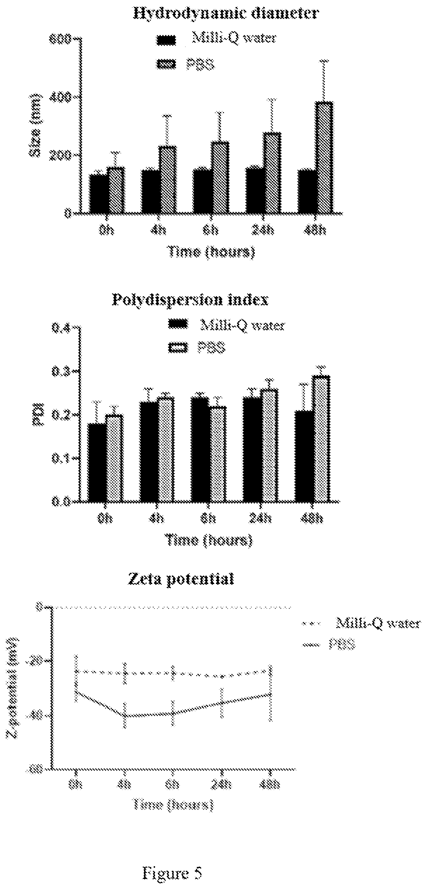

The stability study performed for 48 h at 37 °C showed a good colloidal stability for PLGA/DE-DOPE/miRNA nanoparticles in Milli-Q water, while incubation in PBS led to a slight increase in the size and PDI at 48 h, even if not significant (Figure 5). The zeta potential maintains a negative value in Milli-Q water (-23 mV) remaining constant, while the presence of PBS determines a slight decrease of the zeta potential at 48 h (-32 mV) (Figure 5). Therefore, in this case, interactions between the ions present in the solution and nanoparticles were hypothesized, affecting size after 48 h. Furthermore, as suggested by standard deviation (DS) values, interaction with ions by PLGA/DE-DOPE/miRNA nanoparticle suspensions in PBS likely influenced the repeatability of DLS analysis.

### g) Storage stability at 37 °C in Milli-Q water and DMEM+FBS up to 48 h

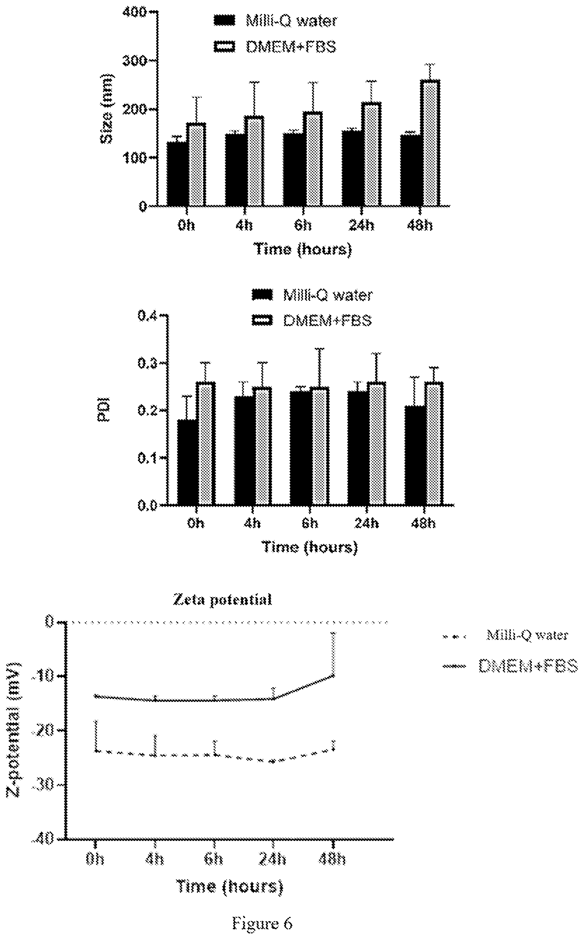

Finally, slight variations of the average size and zeta potential of PLGA/DE-DOPE/miRNA nanoparticles were observed after their incubation for 48 hours in cell culture medium (DMEM+FBS), compared to their incubation in Milli-Q water (Figure 6). The initial average hydrodynamic size of PLGA/DE-DOPE/miRNA nanoparticles in DMEM+FBS was 172 nm and increased to 260 nm after 48 hours incubation. The zeta potential value of nanoparticles in DMEM+FBS increased to -10 mV, remaining stable throughout the study period (Figure 6). Such variations could be the result of an interaction of NP surface with serum proteins. However, also by virtue of PDI values (0.25-0.26) measured after 48 h incubation, it was found that PLGA/DE-DOPE/miRNA nanoparticles maintain good colloidal stability in DMEM+FBS.

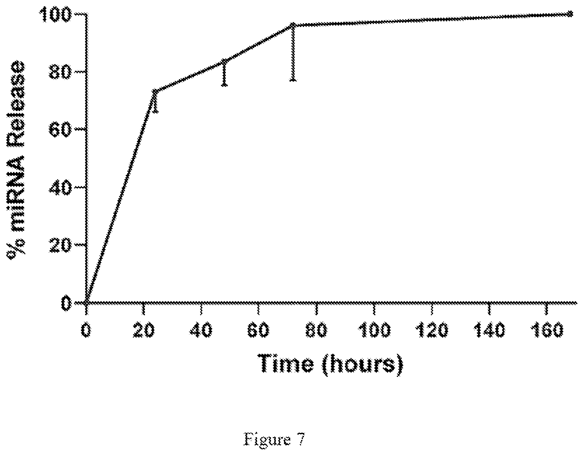

PLGA/DE-DOPE/negmiR nanoparticles in static conditions do not release miRNA at 37 °C in Milli-Q water, showing protection of the oligonucleotide agent. In dynamic conditions at 37 °C instead, PLGA/DE-DOPE/negmiR nanoparticles showed a gradual delivery reaching a release value of 73% after 24 h, and a total release after 7 days (Figure 7). In contrast, DE-DOPE/negmiR particles show a complete release within 24 hours under static conditions (data not shown). The profile obtained in Figure 7 reflects the well-known properties of PLGA as a material capable of protecting drugs from degradation and ensuring controlled release kinetics.

### h) In vitro efficacy assays: uptake, cytotoxicity, miRNA-1 expression, and target downregulation (Twf1)

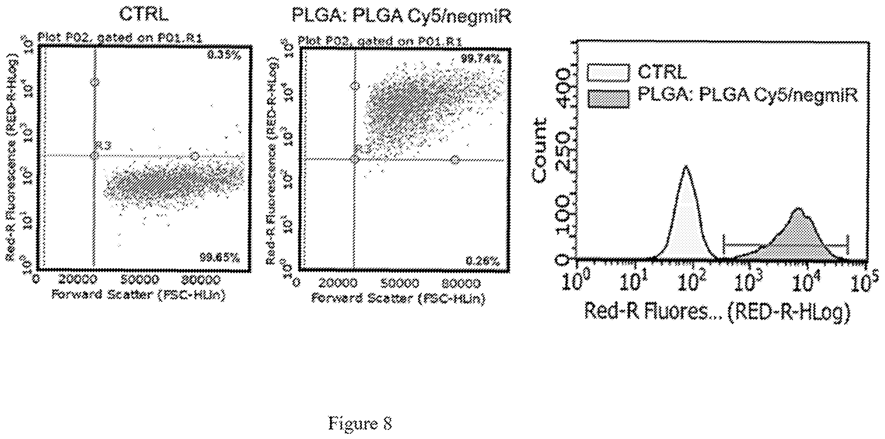

miRNA is efficiently delivered into the cells as demonstrated by cellular assays (using adult human atrial cardiac fibroblasts, AHCFs) through the evaluation of the cellular uptake efficiency of fluorescent hybrid polymeric nanoparticles containing PLGA labelled with Cyanine-5 (cytofluorimetric analysis). After 24 hours from the beginning of the transfection treatment, 99% of the cells was found positive for Cyanine-5, highlighting the efficacy of nanoparticle internalization by the cells (Figure 8).

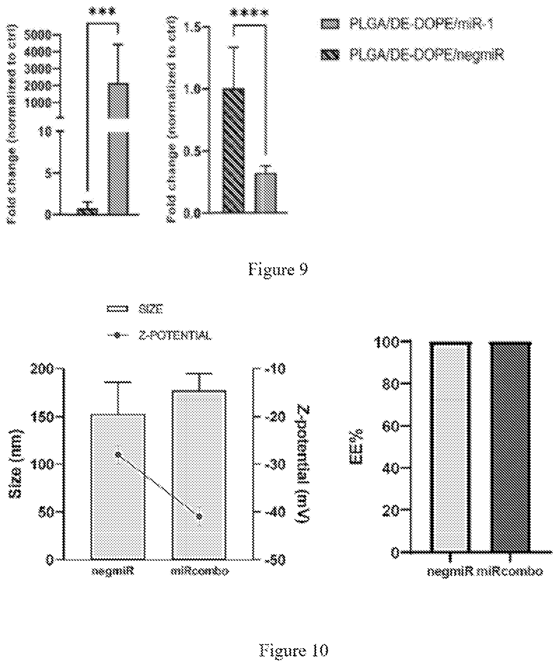

Further cell tests were performed by transfecting AHCFs with hybrid polymeric nanoparticles loaded with miR-1 or control negmiR for 24 hours. For performance comparison, the same cells were transfected with DE-DOPE/miRNA lipidic particles. As assessed by Droplet Digital PCR (ddPCR), miR-1 expression increased significantly using the two types of nanoparticles (lipidic or hybrid) loaded with miR-1 respect to controls (Figure 9: left image). Furthermore, Twf1 downregulation was similar for the two experimental cases of cell transfection with lipidic or hybrid particles releasing miR-1 (Figure 9: right image).

### i) Encapsulation of miRcombo: hydrodynamic size and Z-potential

4 miRNAs (miRcombo) were encapsulated into the hybrid nanoparticles, assessing their physicochemical characteristics. Encapsulation of miRcombo does not change the hydrodynamic size, Z-potential, PDI, and encapsulation efficiency of nanoparticles, compared to the case of single miRNA encapsulation, as shown by data in Table 2.

**Table 2. Characteristics of PLGA/DE-DOPE/miRcombo nanoparticles assessed by DLS analysis.**

| **Nanoparticles** | **Size (nm)** | **PDI** | **Z-potential (mV)** | **EE%** |
|---|---|---|---|---|
| **PLGA/DE-DOPE/miRcombo** | 177 ± 18 | 0.28 ± 0.02 | -41 ± 2 | **99.0 ± 0.2** |

In Figure 10 properties of the hybrid particles loaded with a single miRNA (NegmiR) are compared with those of the hybrid nanoparticles encapsulating miRcombo (4 miRNAs, with a total dose equal to the NegmiR content of the other particles), clearly showing the same characteristics of hydrodynamic size, Z-potential, and PDI.

### j) DLS analysis of PLGA/DE-DOPE/miRcombo nanoparticles incubated in DMEM+FBS

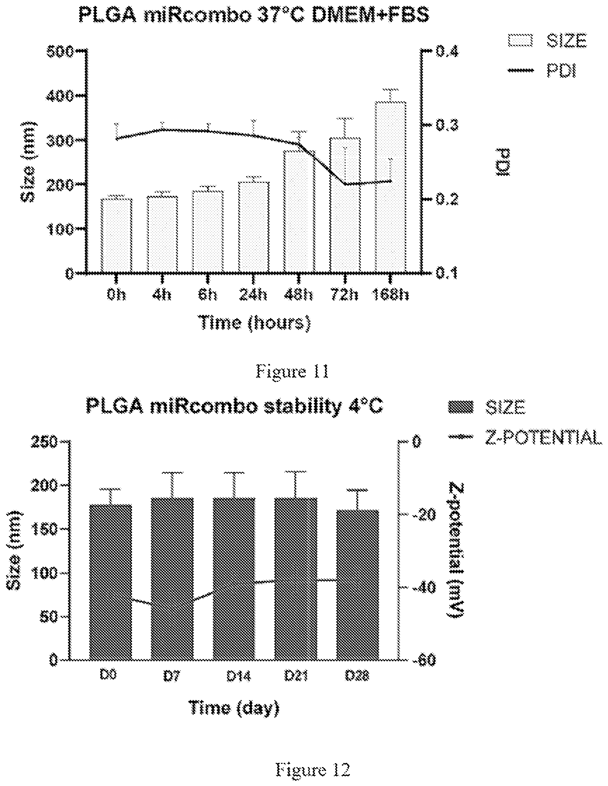

The stability in culture medium (DMEM+FBS) of PLGA/DE-DOPE/miRcombo nanoparticles is similar to that of PLGA/DE-DOPE/negmiR nanoparticles (Figure 11). After 48 h incubation, slight variations in the average size of PLGA/DE-DOPE/miRcombo nanoparticles were observed. The initial average hydrodynamic size of PLGA/DE-DOPE/miRcombo nanoparticles in DMEM+FBS was 167 nm and increased up to 275 nm after 48 h incubation. Moreover, further increase in the hydrodynamic diameter was measured after 7 days (410 nm) due to possible formation of a protein corona. The zeta potential of the nanoparticles in DMEM+FBS was -10 mV (therefore higher than the value in Milli-Q water), remaining stable throughout the study period. This variation could be the result of an interaction of the surface of the NPs with serum proteins. Finally, PDI values after 48 hours incubation were stable, thus demonstrating that PLGA/DE-DOPE/miRcombo nanoparticles retain a good colloidal stability in DMEM+FBS during the transfection period (24 hours), with the possibility to perform *in vitro* transfection even for up to 48 h.

### k) Stability of the hybrid polymeric nanoparticles containing miRcombo in storage conditions (4 °C, Milli-O water)

The hydrodynamic diameter and zeta potential of PLGA/DE-DOPE/miRcombo nanoparticles remain stable up to 28 days incubation at 4 °C in Milli-Q water (Figure 12).

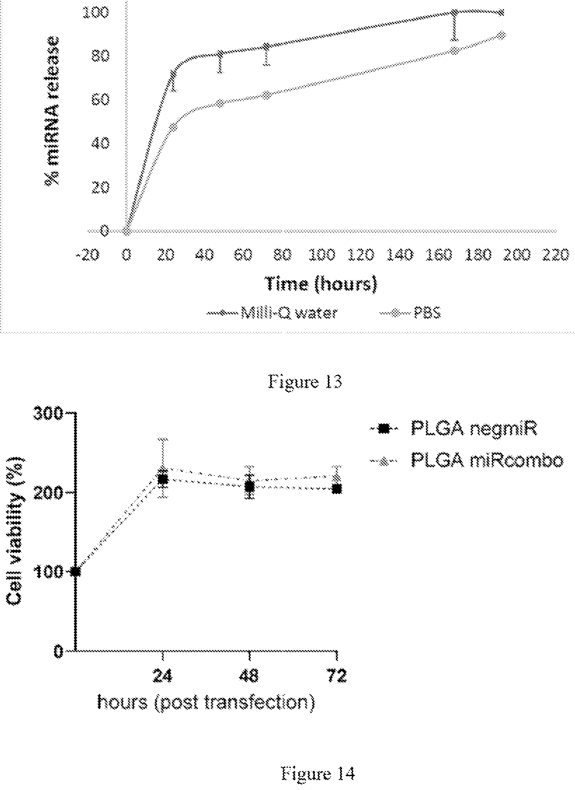

The *in vitro* release of miRcombo in dynamic conditions in Milli-Q water at 37 °C is completed in 168 hours (7 days), similarly to the release of NegmiR (Figure 7, Figure 13). After 24 hours, the release is about 7% and then increases slowly up to 7 days.

Instead, in physiological conditions (PBS), the release is more gradual, reaching 90% after 192 hours (8 days), probably due to the surface adsorption of cations from PBS, which contribute to slow down the release of the miRNAs (negatively charged).

### l) Biocompatibility

The nanoparticles loaded with miRcombo or with negmiR are also biocompatible with AHCFs, as demonstrated by resazurin assay performed for up to 72 hours from the beginning of transfection (Figure 14).

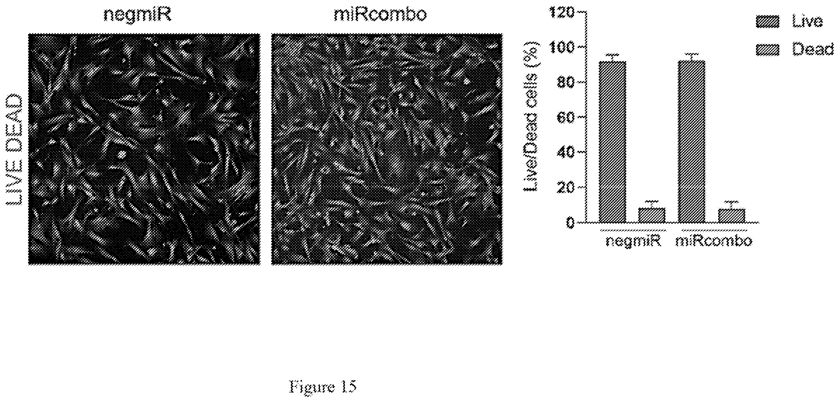

Live/Dead assay, performed on AHCFs after 24 hours transfection with nanoparticles, loaded with negmiR or miRcombo (with the same dose used for biocompatibility tests) and subsequent cell culture up to 72 hours showed cell viability of 95% (Figure 15).

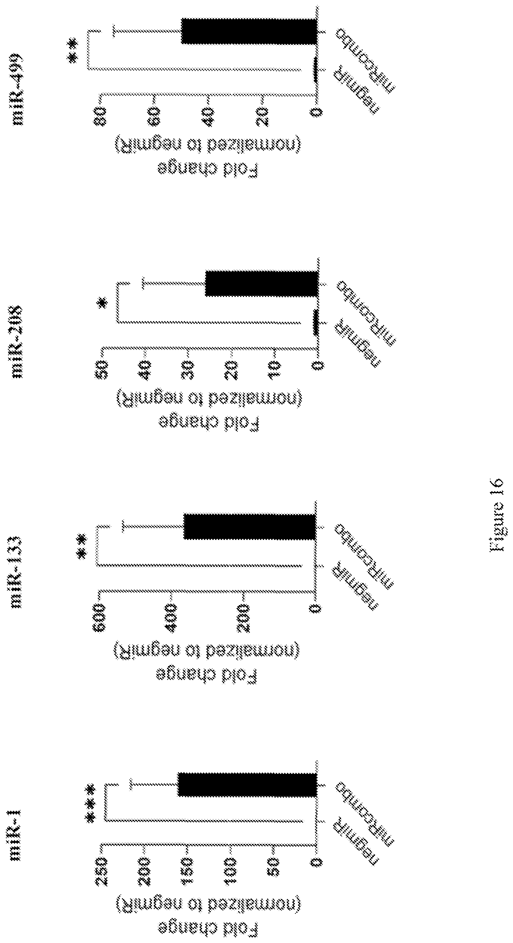

Furthermore, miRcombo is released efficiently into the cells as demonstrated by cellular assays with the evaluation of the presence of single microRNAs by ddPCR analysis at 48 hours from the transfection (Figure 16).

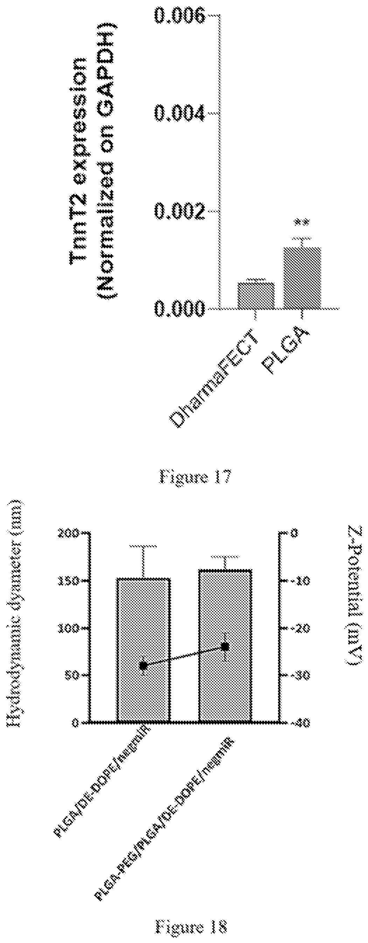

The evaluation of the expression of the cardiac marker TNNT2 at 15 days culture (by ddPCR analysis) showed the efficacy of the administration of miRcombo by the hybrid PLGA/DE-DOPE/miRNA nanoparticles, since it highlighted the cellular trans-differentiation mediated by miRcombo. Trans-differentiation is higher with respect to the commercial transfectant DharmaFECT (Figure 17).

### m) PLGA/PLGA-PEG/DE-DOPE/miRNA nanoparticles: hydrodynamic size and Z-potential

The hybrid nanoparticles containing negmiR were also prepared using a polymeric mixture of PLGA and PLGA-PEG with a weight ratio between the components equal to 50:50, instead of PLGA alone. The resulting nanoparticles did not show variations in hydrodynamic size, Z-potential, PDI, and encapsulation efficacy, compared to PLGA/DE-DOPE/negmiR nanoparticles, as derived from data reported in Table 3.

**Table 3. Characteristics of the PLGA-PEG/PLGA/DE-DOPE/miRNA nanoparticles assessed by DLS analysis.**

| **Nanoparticles** | **Size (nm)** | **PDI** | **Z-potential (mV)** | **EE%** |
|---|---|---|---|---|
| **PLGA/DE-DOPE/miRcombo** | 162 ± 12 | 0.24 ± 0.03 | -24 ± 3 | **99.0 ± 0.2** |

In Figure 18 the properties of hybrid PLGA/DE-DOPE/miRNA particles are compared to PLGA-PEG/PLGA/DE-DOPE/miRNA hybrid nanoparticles which clearly show the same characteristics of hydrodynamic diameter, Z-potential, and PDI.

### n) Surface functionalization with a model molecule as a ligand (Immunoglobulin G - IgG): hydrodynamic size and Z-potential

The hybrid nanoparticles were functionalized with IgG, as model molecule for a ligand (IgG/PLGA-PEG/PLGA/DE-DOPE/miRNA). The used PLGA-PEG copolymer shows a terminal azido group which is used for conjugation. The IgG molecule was pre-modified to expose an alkyne group. The PLGA-PEG/PLGA/DE-DOPE/miRNA nanoparticles were incubated in a solution containing the IgG-modified molecule, in the presence of copper as a catalyst.

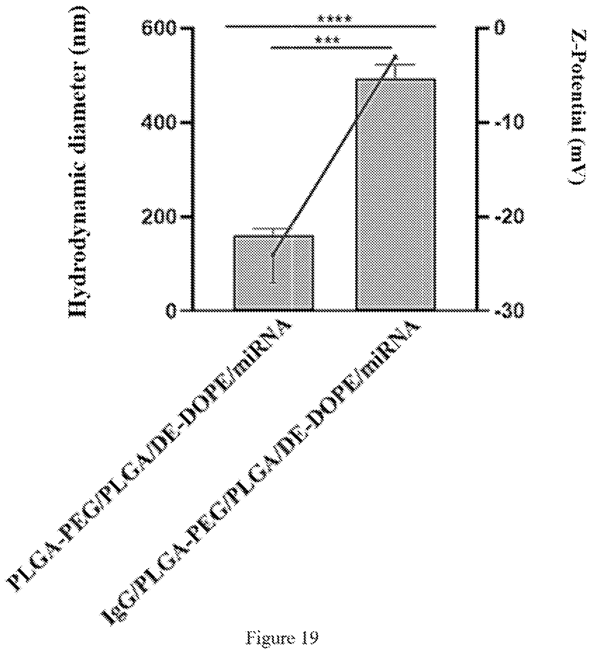

The functionalized hybrid nanoparticles were characterized in terms of hydrodynamic size, PDI, and Z-potential by DLS analysis. As shown in Figure 19, the hybrid IgG/PLGA-PEG/PLGA/DE-DOPE/miRNA nanoparticles show an increase of the hydrodynamic diameter (494 ± 29 nm) and an increase of the Z-potential (-3 ± 0.03 mV) related to the presence of IgG on the surface.

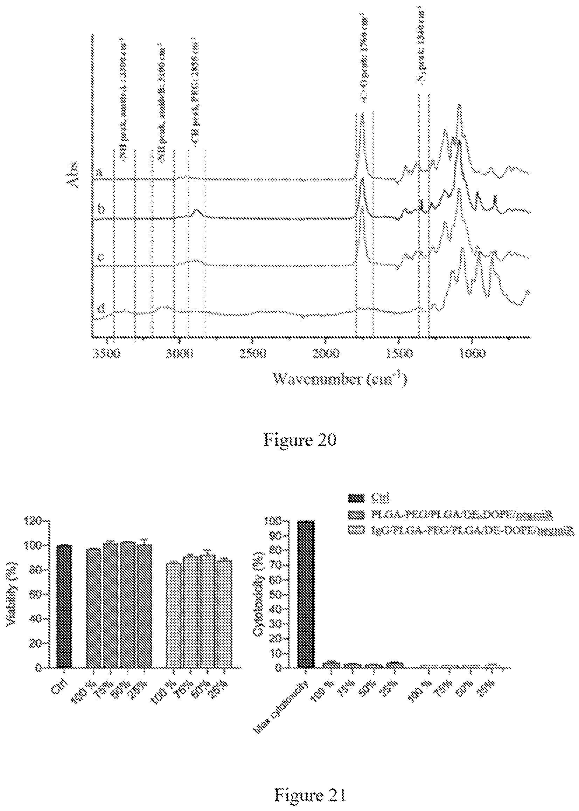

In Figure 20, the FTIR-ATR spectrum of IgG/PLGA-PEG/PLGA/DE-DOPE/miRNA nanoparticles shows absorption bands at 3300 cm⁻¹ and 3100 cm⁻¹ suggesting the presence of IgG on the surface. Absorption bands due to C-H stretching of methylene groups (mainly attributed to PEG) and C=O stretching of ester groups in polyesters and absorption bands of azido groups, present in FTIR-ATR spectrum of PLGA-PEG/PLGA/DE-DOPE/miRNA nanoparticles are significatively attenuated in the spectrum of nanoparticles functionalized with IgG, confirming the presence of IgG on NP surface.

### o) In vitro assays: viability and cytotoxicity of PLGA-PEG/PLGA/DE-DOPE/negmiR and IgG/PLGA-PEG/PLGA/DE-DOPE/ negmiR nanoparticles

Cytocompatibility tests were performed by transfecting AHCFs with the PLGA-PEG/PLGA/DE_DOPE/negmiR and IgG/PLGA-PEG/PLGA/DE-DOPE/negmiR polymeric nanoparticles at different concentrations (25, 18.75, 12.5, and 6.25 nM of miRNA) for 24 hours. Both the nanoparticle formulations resulted in high cellular viability and low cellular cytotoxicity (Figure 21) confirming the cytocompatibility of the nanoparticles.

### p) PLA/DE-DOPE/miRNA nanoparticles: hydrodynamic size and Z-potential

The hybrid nanoparticles were prepared also using another polymer, specifically PLA. The resulting nanoparticles did not show variations in terms of hydrodynamic size, Z-potential, PDI, and encapsulation efficacy, respect to PLGA/DE-DOPE/negmiR nanoparticles, as showed from data reported in Table 4.

**Table 4. Characteristics of the PLA/DE-DOPE/miRNA nanoparticles assessed by DLS analysis.**

| **Nanoparticles** | **Size (nm)** | **PDI** | **Z-potential (mV)** | **EE%** |
|---|---|---|---|---|
| **PLA/DE-DOPE/negmiR** | 160 ± 40 | 0.23 ± 0.07 | -18 ± 3 | **99.0 ± 0.2** |

### q) Zein/DE-DOPE/miRNA nanoparticles: hydrodynamic size and Z-potential

Hybrid nanoparticles were also prepared using another polymer, specifically zein. Resulting nanoparticles did not show variations in terms of hydrodynamic size, Z-potential, PDI, and encapsulation efficiency, respect to PLGA/DE-DOPE/negmiR nanoparticles, as shown from data reported in Table 5.

**Table 5. Characteristics of the zein/DE-DOPE/miRNA nanoparticles assessed by DLS analysis.**

| **Nanoparticles** | **Size (nm)** | **PDI** | **Z-potential (mV)** | **EE%** |
|---|---|---|---|---|
| **Zein/DE-DOPE/negmiR** | 177 ± 30 | 0.20 ± 0.04 | -17 ± 4 | **97.0 ± 2** |

## Claims

1. Hybrid nanoparticles comprising a polymeric component encapsulating a lipid core comprising cationic liposomes or lipoplexes comprising a cationic lipid and a helper lipid, wherein:
- the polymeric component constitutes at least the 80% by weight with respect to the total material constituting the nanoparticles;
- the cationic lipid is selected from [2-(2,3-didodecyloxypropyl)-hydroxyethyl] ammonium bromide, 1,2-di-O-octadecenyl-3-trimethylammonium propane, 1,2-dioleoyloxy-3-[trimethylammonium]-propane, dimethyl-dioctadecyl ammonium bromide, cetyl-trimethyl ammonium bromide, 2,3-dioleyloxy-N-[2-(sperminecarboxamido)ethyl]-N,N-dimethyl-1-propane amino trifluoroacetate, bis-guanidinium-tren-cholesterol;
- the weight ratio between the cationic lipid and the helper lipid is 50:50,
said hybrid nanoparticles obtainable by nano-precipitation of a solution of the polymeric component in a dispersion of the cationic liposomes or lipoplexes.

2. Nanoparticles according to claim 1 wherein the polymeric component is a single polymer or a mixture of polymers.

3. Nanoparticles according to claim 1 or 2 wherein the polymeric component is selected from polyesters, poly(hydroxy alkanoates), poly(propylene oxides), methacrylic polymers or copolymers, poly(cyanoacrylates), polyurethanes, their copolymers with PEG and/or with poly(ethylene oxide), possibly modified natural polymers.

4. Nanoparticles according to claims 2 and 3 wherein the polymeric component is selected from poly(lactic-co-glycolic acid), poly(glycolic acid), poly(lactic acid), polycaprolactone, poly(lactide-co-caprolactone), poly(hydroxy butyrate), poly(hydroxy butyrate-co-hydroxy valerate) copolymer, poly(propylene oxide), poly(methyl methacrylate), poly(ethyl methacrylate), poly(butyl methacrylate), zein, cellulose, lignin, starch, butyl glyceryl pectin.

5. Nanoparticles according to any one of claims 1 to 4 wherein the cationic lipid comprises a hydrophilic head with a permanent positive charge having ammonium groups.

6. Nanoparticles according to one or more of claims 1 to 5 wherein the helper lipid is selected from phosphatidylethanolamine, phosphatidylcholine, cholesterol, or mixtures thereof.

7. Nanoparticles according to one or more of claims 1 to 6 wherein the lipoplexes include polynucleotides selected among microRNAs, siRNAs, mRNAs, plasmid DNAs, and antisense oligonucleotides.

8. Nanoparticles according to one or more of claims 1 to 7 comprising also hydrophobic drugs.

9. Nanoparticles according to one or more of claims 1 to 8 functionalized with one or more ligands.

10. Nanoparticles according to claim 9 wherein the ligand is an antibody, antigen-binding antibody fragment (Fab), aptamer, peptide, carbohydrate, small molecules, or a combination of the foregoing.

11. A method for the preparation of the nanoparticles of claims 1-10 comprising:
a) The preparation of an aqueous dispersion of liposomes or lipoplexes;
b) The dropping of a solution of the polymeric component in an organic solvent miscible with water within an aqueous dispersion of the liposomes or lipoplexes under stirring;
c) Removal of the organic solvent.

12. A method according to claim 11 wherein the nano-precipitation occurs in a microfluidic device.

13. A method according to claim 11 or 12 wherein emulsifiers or surfactants are not added.

14. Hybrid nanoparticles of claims 1 to 10 for use in regenerative medicine, cellular reprogramming, treatment of tumors, treatment of neurodegenerative diseases, and as vaccine.

## Patentansprüche

1. Hybride Nanopartikel, umfassend eine polymere Komponente, die einen Lipidkern einkapselt, umfassend kationische Liposomen oder Lipoplexe, umfassend ein kationisches Lipid und ein Helferlipid, wobei:
- die polymere Komponente mindestens 80 Gew.-% des Gesamtmaterials der Nanopartikel ausmacht;
- das kationische Lipid ausgewählt ist aus [2-(2,3-Didodecyloxypropyl)-hydroxyethyl]ammoniumbromid, 1,2-Di-O-octadecenyl-3-trimethylammoniumpropan, 1,2-Dioleoyloxy-3-[trimethylammonium]-propan, Dimethyldioctadecylammoniumbromid, Cetyltrimethylammoniumbromid, 2,3-Dioleyloxy-N-[2-(spermincarboxamido)ethyl]-N,N-dimethyl-1-propanoaminotrifluoracetat, Bisguanidinium-trencholesterin ausgewählt wird;
- das Gewichtsverhältnis zwischen dem kationischen Lipid und dem Helferlipid 50:50 beträgt,
wobei die hybriden Nanopartikel durch Nanopräzipitation einer Lösung der polymeren Komponente in einer Dispersion der kationischen Liposomen oder Lipoplexe erhältlich sind.

2. Nanopartikel gemäß Anspruch 1, wobei die polymere Komponente ein einzelnes Polymer oder eine Mischung von Polymeren ist.

3. Nanopartikel gemäß Anspruch 1 oder 2, wobei die polymere Komponente ausgewählt ist aus Polyestern, Poly(hydroxyalkanoaten), Poly(propylenoxiden), Methacrylpolymeren oder -copolymeren, Poly(cyanoacrylaten), Polyurethanen, deren Copolymeren mit PEG und/oder mit Poly(ethylenoxid), gegebenenfalls modifizierten natürlichen Polymeren.

4. Nanopartikel gemäß den Ansprüchen 2 und 3, wobei die polymere Komponente ausgewählt ist aus Poly(milchsäure-co-glykolsäure), Poly(glykolsäure), Poly(milchsäure), Polycaprolacton, Poly(lactid-co-caprolacton), Poly(hydroxybutyrat), Poly(hydroxybutyrat-co-hydroxyvalerat)-Copolymer, Poly(propylenoxid), Poly(methylmethacrylat), Poly(ethylmethacrylat), Poly(butylmethacrylat), Zein, Cellulose, Lignin, Stärke, Butylglycerylpektin.

5. Nanopartikel gemäß einem der Ansprüche 1 bis 4, wobei das kationische Lipid einen hydrophilen Kopf mit einer permanenten positiven Ladung mit Ammoniumgruppen umfasst.

6. Nanopartikel gemäß einem oder mehreren der Ansprüche 1 bis 5, wobei das Helferlipid aus Phosphatidylethanolamin, Phosphatidylcholin, Cholesterin oder Mischungen davon ausgewählt ist.

7. Nanopartikel gemäß einem oder mehreren der Ansprüche 1 bis 6, wobei die Lipoplexe Polynukleotide umfassen, ausgewählt aus microRNAs, siRNAs, mRNAs, Plasmid-DNAs und Antisense-Oligonukleotiden ausgewählt.

8. Nanopartikel gemäß einem oder mehreren der Ansprüche 1 bis 7, des Weiteren umfassend hydrophobe Arzneimittel.

9. Nanopartikel gemäß einem oder mehreren der Ansprüche 1 bis 8, funktionalisiert mit einem oder mehreren Liganden.

10. Nanopartikel gemäß Anspruch 9, wobei der Ligand ein Antikörper, ein Antigen-bindendes Antikörperfragment (Fab), ein Aptamer, ein Peptid, ein Kohlenhydrat, kleine Moleküle oder eine Kombination der vorgenannten Stoffe ist.

11. Verfahren zur Herstellung der Nanopartikel gemäß den Ansprüchen 1 bis 10, umfassend:
a) die Herstellung einer wässrigen Dispersion von Liposomen oder Lipoplexen;
b) das Einbringen einer Lösung der polymeren Komponente in einem mit Wasser mischbaren organischen Lösungsmittel in eine wässrige Dispersion der Liposomen oder Lipoplexe unter Rühren;
c) Entfernen des organischen Lösungsmittels.

12. Verfahren nach Anspruch 11, wobei die Nanopräzipitation in einer mikrofluidischen Vorrichtung stattfindet.

13. Verfahren nach Anspruch 11 oder 12, wobei keine Emulgatoren oder Tenside hinzugefügt werden.

14. Hybrid-Nanopartikel gemäß den Ansprüchen 1 bis 10 zur Verwendung in der regenerativen Medizin, der zellulären Reprogrammierung, der Behandlung von Tumoren, der Behandlung neurodegenerativer Erkrankungen und als Impfstoff.

## Revendications

1. Nanoparticules hybrides comprenant un composant polymère encapsulant un noyau lipidique comprenant des liposomes ou lipoplexes cationiques comprenant un lipide cationique et un lipide auxiliaire :
- le composant polymère constituant au moins 80% en poids par rapport à la matière totale constituant les nanoparticules ;
- le lipide cationique étant choisi parmi le bromure de [2-(2,3-didodécyloxypropyl)-hydroxyéthyl]ammonium, le 1,2-di-O-octadécényl-3-triméthylammonium-propane, le 1,2-dioléoyloxy-3-[triméthylammonium]-propane, le bromure de diméthyldioctadécylammonium, le bromure de cétyltriméthylammonium, l'aminotrifluoroacétate de 2,3-dioléyloxy-N[2-(sperminecarboxamido)éthyl]-N,N-diméthyl-1-propane, le bis-guanidinium-tren-cholestérol ;
- le rapport pondéral entre le lipide cationique et le lipide auxiliaire valant 50:50, lesdites nanoparticules hybrides pouvant être obtenues par nanoprécipitation d'une solution du composant polymère dans une dispersion des liposomes ou lipoplexes cationiques.

2. Nanoparticules selon la revendication 1, le composant polymère étant un polymère unique ou un mélange de polymères.

3. Nanoparticules selon la revendication 1 ou 2, le composant polymère étant choisi parmi les polyesters, les poly(hydroxyalcanoates), les poly(oxydes de propylène), les polymères ou copolymères méthacryliques, les poly(cyanoacrylates), les polyuréthanes, leurs copolymères avec du PEG et/ou avec du poly(oxyde d'éthylène), les polymères naturels éventuellement modifiés.

4. Nanoparticules selon les revendications 2 et 3, le composant polymère étant choisi parmi le poly(acide lactique-co-acide glycolique), le poly(acide glycolique), le poly(acide lactique), la polycaprolactone, la poly(lactide-co-caprolactone), le poly(hydroxybutyrate), le copolymère de poly(hydroxybutyrate-co-hydroxyvalérate), le poly(oxyde de propylène), le poly(méthacrylate de méthyle), le poly(méthacrylate d'éthyle), le poly(méthacrylate de butyle), la zéine, la cellulose, la lignine, l'amidon, la butylglycérylpectine.

5. Nanoparticules selon l'une quelconque des revendications 1 à 4, le lipide cationique comprenant une tête hydrophile présentant une charge positive permanente présentant des groupes ammonium.

6. Nanoparticules selon l'une ou plusieurs des revendications 1 à 5, le lipide auxiliaire étant choisi parmi la phosphatidyléthanolamine, la phosphatidylcholine, le cholestérol ou leurs mélanges.

7. Nanoparticules selon l'une ou plusieurs des revendications 1 à 6, les lipoplexes comprenant des polynucléotides choisis par les microARN, les ARNsi, les ARNm, les ADN de plasmide et les oligonucléotides antisens.

8. Nanoparticules selon l'une ou plusieurs des revendications 1 à 7, comprenant également des médicaments hydrophobes.

9. Nanoparticules selon l'une ou plusieurs des revendications 1 à 8, fonctionnalisées par un ou plusieurs ligands.

10. Nanoparticules selon la revendication 9, le ligand étant un anticorps, un fragment d'un anticorps se liant à l'antigène (Fab), un aptamère, un peptide, un glucide, de petites molécules ou une combinaison des substances susmentionnées.

11. Procédé pour la préparation des nanoparticules selon les revendications 1-10, comprenant :
a) la préparation d'une dispersion aqueuse de liposomes ou lipoplexes ;
b) l'ajout goutte à goutte d'une solution du composant polymère dans un solvant organique miscible à l'eau dans une dispersion aqueuse des liposomes ou lipoplexes sous agitation ;
c) l'élimination du solvant organique.

12. Procédé selon la revendication 11, la nanoprécipitation se produisant dans un dispositif microfluidique.

13. Procédé selon la revendication 11 ou 12, des émulsifiants ou des tensioactifs n'étant pas ajoutés.

14. Nanoparticules hybrides selon les revendications 1 à 10, pour l'utilisation en médecine régénérative, en reprogrammation cellulaire, en traitement de tumeurs, en traitement de maladies neurodégénératives et comme vaccin.
